# FASCICULE DE BREVET EUROPEEN

(11) **EP 4 264 257 B1**
(45) Date de publication et mention de la délivrance du brevet: **19.03.2025**
(21) Numéro de dépôt: 21848010.1
(22) Date de dépôt: 15.12.2021
(51) Int. Cl.: G01N 33/00, F24F 11/62, F24F 110/50

(54) **DSIPOSITIF DE SUIVI D'UN COMPOSANT DE L'AIR**
VORRICHTUNG ZUR ÜBERWACHUNG EINER KOMPONENTE DER LUFT
DEVICE FOR MONITORING A COMPONENT OF THE AIR

(30) Priorité: 17.12.2020 FR 2013434
(43) Date de publication de la demande: 25.10.2023
(73) Titulaire: SEB S.A., 69130 Ecully (FR)
(72) Inventeur: GAUTHIER, Sébastien, 69134 Ecully Cedex (FR); ABEDINI, Farhad, 69134 Ecully Cedex (FR)
(74) Mandataire: Germain Maureau
(86) Numéro de dépôt international: PCT/FR2021/052338
(87) Numéro de publication internationale: WO 2022/129787

(56) Documents cités:
- ILYAS EUSHAY BIN ET AL: "Virtual Sensor Creation to Replace Faulty Sensors Using Automated Machine Learning Techniques", 2020 GLOBAL INTERNET OF THINGS SUMMIT (GIOTS), IEEE, 3 June 2020 (2020-06-03), pages 1 - 6, XP033782451, DOI: 10.1109/GIOTS49054.2020.9119681
- ZAIDAN MARTHA ARBAYANI ET AL: "Intelligent Calibration and Virtual Sensing for Integrated Low-Cost Air Quality Sensors", IEEE SENSORS JOURNAL, IEEE SERVICE CENTER, NEW YORK, NY, US, vol. 20, no. 22, 17 July 2020 (2020-07-17), pages 13638 - 13652, XP011815186, ISSN: 1530-437X, [retrieved on 20201015], DOI: 10.1109/JSEN.2020.3010316
- ZAIDAN MARTHA A. ET AL: "Mutual Information Input Selector and Probabilistic Machine Learning Utilisation for Air Pollution Proxies", APPLIED SCIENCES, vol. 9, no. 20, 22 October 2019 (2019-10-22), pages 4475, XP055831598, DOI: 10.3390/app9204475
- LEIDINGER M. ET AL: "Selective detection of hazardous VOCs for indoor air quality applications using a virtual gas sensor array", JOURNAL OF SENSORS AND SENSOR SYSTEMS, vol. 3, no. 2, 21 October 2014 (2014-10-21), pages 253 - 263, XP055831596, DOI: 10.5194/jsss-3-253-2014
- MATUSOWSKY MICHAEL ET AL: "Data Imputation in Wireless Sensor Networks Using a Machine Learning-Based Virtual Sensor", JOURNAL OF SENSOR AND ACTUATOR NETWORKS, vol. 9, no. 2, 27 May 2020 (2020-05-27), pages 25, XP055831597, DOI: 10.3390/jsan9020025

## Description

La présente invention concerne de manière générale un dispositif de suivi d'un composant de l'air, permettant notamment de mesurer la concentration dans l'air du composant au cours du temps.

Il est connu dans l'art antérieur des dispositifs de suivi de la qualité de l'air permettant de mesurer un ou plusieurs paramètres de l'air ambiant comme un taux d'humidité ou une concentration d'un ou plusieurs composants de l'air, tel que des particules ou des composés organiques volatils.

En contrepartie, ces dispositifs peuvent être couteux, complexes à mettre en œuvre et donc inadaptés à une utilisation domestique. Par exemple, ils peuvent nécessiter l'utilisation de capteur spécifique, comme des capteurs consommables c'est-à-dire à durée de vie limitée. De tels capteurs peuvent présenter une précision supérieure, permettre une absence d'étalonnage une fois installée ou bien détecter des composants de l'air spécifique non détectables avec d'autres capteurs.

ILYAS EUSHAY BIN ET AL: "Virtual Sensor Creation to Replace Faulty Sensors Using Automated Machine Learning Techniques", 2020 GLOBAL INTERNET OF THINGS SUMMIT (GIOTS), IEEE, 3 juin 2020, pages 1-6, XP033782451, DOI: 10.11091 GIOTS49054.2020.9119681 divulgue le remplacement d'un capteur défectueux par un capteur virtuel.

ZAIDAN MARTHA ARBAYANI ET AL: "Intelligent Calibration and Virtual Sensing for Integrated Low-Cost Air Quality Sensors", IEEE SENSORS JOURNAL, vol. 20, no. 22, 17 juillet 2020, pages 13638-13652, XP011815186, DOI: 10.1109/JSEN.2020.3010316 divulgue l'utilisation d'un capteur virtuel dans le contexte de mesure de la qualité de l'air.

Un but de la présente invention est de répondre aux inconvénients des documents de l'art antérieur mentionnés ci-dessus et en particulier, tout d'abord, de proposer un dispositif de suivi d'un composant de l'air peut couteux et pouvant mesurer et/ou estimer des concentrations dans l'air de composants spécifiques.

Pour cela, un premier aspect de l'invention concerne un dispositif de suivi d'un composant de l'air, le dispositif comprenant une pluralité de capteurs configurés pour acquérir des valeurs de paramètres environnementaux,
caractérisé en ce que le dispositif comprend en outre :
- un capteur consommable configuré pour acquérir des valeurs de concentration dudit composant, le capteur consommable ayant une durée de vie prédéterminée et
- un calculateur configuré pour :
   - dans une phase d'apprentissage, établir un modèle de la concentration dudit composant en établissant une corrélation entre des valeurs de concentration dudit composant acquises par le capteur consommable et des valeurs des paramètres environnementaux acquises par la pluralité de capteurs et
   - dans une phase de suivi, estimer la concentration dudit composant en utilisant le modèle établi et des valeurs des paramètres environnementaux acquises par la pluralité de capteurs.

Ainsi, le présent dispositif de suivi permet de suivre, c'est-à-dire de mesurer puis d'estimer la concentration d'un composant de l'air qui ne serait pas possible d'obtenir sans capteur consommable et sans renouveler ce capteur consommable régulièrement.

De manière avantageuse, le calculateur peut être configuré pour réaliser la phase de suivi au moins à partir du moment où la durée de vie du capteur consommable est expirée ou à partir du moment où le capteur consommable est déconnecté du dispositif. Ainsi, le capteur consommable peut être utilisé intégralement avant la réalisation de la phase de suivi ou bien il peut être retiré pour être utilisé ultérieurement.

Avantageusement, le calculateur peut en outre être configuré pour, dans une phase de validation, comparer des valeurs estimées de concentration du composant à des valeurs de concentration du composant acquises par le capteur consommable. Cette phase de validation peut permettre de valider que le modèle établi fournit une estimation précise de la concentration du composant de l'air avant que la durée de vie du capteur consommable ne soit expirée.

Avantageusement, le calculateur peut en outre être configuré pour démarrer une nouvelle phase d'apprentissage ou pour envoyer un message à un utilisateur l'informant de la nécessité de démarrer une nouvelle phase d'apprentissage, si un écart entre des valeurs de concentration du composant estimées et des valeurs de concentration du composant acquises égale ou dépasse un seuil de validité prédéterminé du modèle établi durant la phase de validation.

De manière alternative, la valeur estimée du composant de l'air peut être corrigée par un facteur de correction. Ainsi, les valeurs estimées peuvent gagner en précision et en fiabilité.

Une réalisation particulièrement intéressante consiste en ce que le dispositif puisse comprendre une interface utilisateur et dans lequel le calculateur peut être configuré pour attribuer une durée de vie au modèle établi et, quand la durée de vie du modèle préétabli est expirée, pour démarrer une nouvelle phase d'apprentissage ou pour envoyer un message à un utilisateur sur l'interface utilisateur l'informant de la nécessité de démarrer une nouvelle phase d'apprentissage.

Une telle réalisation peut permettre d'obtenir des valeurs estimées fiables au cours du temps, notamment en tenant compte du fait que l'environnement du dispositif est susceptible de changer au cours du temps, pouvant rendre ainsi le modèle obsolète. Par exemple, la durée de vie du modèle établi est fixée à 3 mois, 6 mois, 1 an, 18 mois, 2 ans ou 3 ans. En outre, l'interface utilisateur peut permettre de fixer directement ou indirectement la durée de vie du modèle, par exemple selon un niveau de fiabilité attendu des valeurs estimées.

Avantageusement, le dispositif peut comprendre en outre un capteur de position ou une interface utilisateur permettant à l'utilisateur de renseigner une position du dispositif, et dans lequel le calculateur peut être configuré pour démarrer une nouvelle phase d'apprentissage en cas de changement de position dudit dispositif ou pour envoyer un message à un utilisateur sur l'interface utilisateur l'informant de la nécessité de démarrer une nouvelle phase d'apprentissage.

Ainsi, un tel capteur de position peut détecter un changement de l'environnement du dispositif, par exemple un déplacement vers un lieu ou un emplacement différent. Par exemple, le calculateur peut détecter une durée de déplacement et démarrer la nouvelle phase d'apprentissage ou envoyer le message à l'utilisateur au-delà d'une durée de déplacement prédéterminée.

Par exemple, une obsolescence du modèle peut être établie sur la base de valeurs des paramètres environnementaux acquis, par exemple en cas de changement important et/ou brusque dans une ou plusieurs valeurs au cours du temps ou par rapport à une ou plusieurs valeurs moyennes correspondantes.

De manière spécialement avantageuse, le dispositif peut comprendre en outre une interface de communication configurée pour recevoir des valeurs des paramètres environnementaux et le calculateur peut être configuré pour, dans la phase d'apprentissage, établir le modèle en établissant également une corrélation avec les valeurs des paramètres environnementaux reçues et, dans la phase de suivi, estimer la concentration dudit composant en utilisant également les valeurs des paramètres environnementaux reçues.

Ces valeurs des paramètres environnementaux peuvent être reçues à partir d'un ou plusieurs ordinateurs dans le nuage et/ou à partir d'un ou plusieurs dispositifs communicants, par exemple des dispositifs domotiques. Les paramètres environnementaux peuvent alors concerner un environnement intérieur comme une luminosité intérieure, une position des volets de fenêtres ou le caractère ouvert ou fermé d'une porte d'entrée ou d'une fenêtre, ou bien un environnement extérieur comme une vitesse et une direction du vent ou une hygrométrie de l'air extérieur. Un ou plusieurs de ces paramètres environnementaux peuvent en outre être obtenus par un modèle tiers, par exemple une situation météorologique et/ou une prévision météorologique liée à la localisation du dispositif.

Par exemple, une obsolescence du modèle peut être établie sur la base de valeurs des paramètres environnementaux reçues, par exemple en cas de changement important dans une ou plusieurs valeurs au cours du temps ou par rapport à une ou plusieurs valeurs moyennes correspondantes.

Un second aspect de l'invention concerne un appareil de traitement de l'air comprenant un dispositif de suivi de composant de l'air selon le premier aspect de l'invention. Cet appareil de traitement de l'air peut être configuré pour traiter l'air d'un environnement intérieur, c'est-à-dire d'un environnement fermé. Préférentiellement, l'appareil de traitement de l'air peut être configuré pour traiter l'air de façon à faire diminuer au moins la concentration du composant de l'air. De manière encore plus avantageuse, l'appareil de traitement de l'air peut permettre de réduire les concentrations d'autres composants de l'air.

Ainsi, un utilisateur peut suivre une amélioration de la qualité de l'air du fait du traitement réalisé par l'appareil de traitement de l'air. En outre, les paramètres environnementaux peuvent comprendre des paramètres liés au dispositif de traitement de l'air, par exemple un degré d'encrassement ou une durée de vie d'un ou de plusieurs filtres, un historique des vitesses de rotation du moteur, un historique des puissance électrique consommé par le moteur.

Avantageusement, un calculateur peut être configuré pour contrôler un paramètre de traitement de l'air en fonction de la concentration estimée du composant. Un tel paramètre peut comprendre un degré d'ouverture d'un volet ou une puissance ou une vitesse de rotation d'un moteur électrique ou d'un ventilateur. De cette façon, le fonctionnement de l'appareil de traitement de l'air peut être optimisé, de façon à réduire la consommation électrique et/ou la consommation des filtres et autres consommables tout en améliorant la qualité de l'air.

Un troisième aspect de l'invention concerne un procédé de suivi d'un composant de l'air comprenant :
a) une phase d'apprentissage incluant les étapes de :
   i. réception de valeurs de paramètres environnementaux acquises par une pluralité de capteurs,
   ii. réception de valeurs de concentrations dudit composant acquises par un capteur consommable spécifique au composant, le capteur consommable ayant une durée de vie prédéterminée,
   iii. établissement d'un modèle de la concentration dudit composant en établissant une corrélation entre les valeurs de concentration dudit composant et les valeurs des paramètres environnementaux réceptionnées ;
b) une phase de suivi du composant de l'air comprenant une estimation de la concentration du composant de l'air sur la base de valeurs des paramètres environnementaux et du modèle établi.

Un tel procédé peut être mis en œuvre par n'importe quel calculateur comprenant une interface de communication. Il peut s'agir d'un calculateur embarqué à un dispositif de suivi du composant de l'air ou à un appareil de traitement de l'air, ou bien un calculateur distant comme un calculateur de téléphone intelligent, d'ardoise ou tablette tactile, d'ordinateur ou encore d'un calculateur situé dans le nuage.

L'utilisation d'un calculateur distant peut permettre de réduire le cout et la complexité d'un dispositif de suivi du composant de l'air en profitant d'une puissance de calcul déjà possédé par l'utilisateur. Par exemple, les capteurs de paramètres environnementaux et le capteur consommable peuvent être logé dans un dispositif de suivi d'un composant de l'air, portatif ou non. En outre, des valeurs de paramètres environnementaux peuvent être reçues à partie de bases de données ou sources publics ou privées.

Avantageusement, le procédé peut comprendre une phase de validation dans laquelle des valeurs estimées de concentration de composant sont comparées à des valeurs de concentration de composant acquises par le capteur consommable.

Un dernier aspect de l'invention concerne un procédé d'affichage de concentrations d'un composant de l'air comprenant :
a) Une phase d'apprentissage incluant les étapes de :
   i. acquisition de valeurs de paramètres environnementaux par une pluralité de capteurs,
   ii. acquisition de valeurs de concentration dudit composant par un capteur consommable spécifique au composant, le capteur consommable ayant une durée de vie prédéterminée,
   iii. communication des valeurs acquises des paramètres environnementaux et de valeurs de concentration dudit composant à un calculateur distant configuré pour établir un modèle de la concentration dudit composant en établissant une corrélation entre les valeurs de concentration dudit composant et les valeurs des paramètres environnementaux réceptionnées,
b) Une phase de suivi du composant de l'air comprenant les étapes de :
   i. acquisition de valeurs de paramètres environnementaux par une pluralité de capteurs,
   ii. communication des valeurs acquises de paramètres environnementaux au calculateur distant,
   iii. réception et affichage de valeurs estimées de la concentration du composant sur la base des valeurs acquises des paramètres environnementaux et du modèle établi à partir du calculateur distant.

Un tel procédé peut être mis en œuvre par un dispositif ne comprenant pas de calculateur mais uniquement les capteurs et le capteur consommable ainsi qu'une interface de communication. Un tel dispositif peut donc être d'une complexité moindre et d'un cout plus accessible. Il peut par exemple s'agir d'un dispositif portatif. En outre, l'étape d'affichage peut être réalisée sur un appareil distant, comme un téléphone intelligent, une tablette tactile ou un ordinateur.

De manière avantageuse pour tous les aspects précités de la présente invention, le composant de l'air peut être le formaldéhyde. Alternativement, il peut s'agir d'un autre composé organique volatil léger, c'est-à-dire comprenant entre deux et cinq atomes de carbone.

De manière également avantageuse et pour tous les aspects de l'invention, les paramètres environnementaux peuvent être choisis parmi une température, un niveau de luminosité, un niveau de bruit, un degré d'humidité, une pression atmosphérique, un indice de réfraction de l'air, une concentration d'ozone, une concentration de dioxyde de carbone, une concentration de monoxyde de carbone, une concentration d'oxyde(s) d'azote, une concentration d'oxyde de soufre, une concentration d'ammoniaque, une concentration de méthane, une concentration de composés organiques volatiles, une concentration de particules et une position géographique.

D'autres caractéristiques et avantages de la présente invention apparaîtront plus clairement à la lecture de la description détaillée qui suit d'un mode de réalisation de l'invention donné à titre d'exemple nullement limitatif et illustré par les dessins annexés, dans lesquels :
la figure 1 représente un schéma de principe du dispositif de suivi d'un composant de l'air selon la présente invention
la figure 2 représente un schéma d'un procédé de suivi d'un composant de l'air selon la présente invention,
la figure 3 représente un schéma de principe d'un autre dispositif de suivi selon la présente invention,
la figure 4 représente un schéma de principe d'un appareil de traitement de l'air selon la présente invention,
la figure 5 représente des courbes de valeurs des paramètres environnementaux et de la concentration du composant de l'air mesurées au cours du temps, de manière à réaliser une phase d'apprentissage puis une phase de validation,
la figure 6 représente une courbe de concentration mesurée du composant de l'air selon la Figure 5, en comparaison d'une courbe de concentrations estimées par le modèle selon la présente invention,
la figure 7 représente d'autres courbes de valeurs des paramètres environnementaux et de la concentration du composant de l'air mesurées au cours du temps, de manière à réaliser une autre phase de validation,
la figure 8 représente la courbe de concentration mesurée du composant de l'air selon la figure 7, en comparaison avec une autre courbe de concentrations estimées par le modèle selon la présente invention.

L'invention concerne généralement un dispositif de suivi de la qualité de l'air ou moniteur d'air intérieur ou extérieur. Une telle invention permet ainsi d'informer un ou plusieurs utilisateurs sur la qualité de l'air présent dans un environnement comme un environnement ouvert tel qu'un parc, une rue, une terrasse ou préférentiellement un environnement fermé comme une pièce, un appartement ou un bureau.

Ainsi, le terme de « concentration » est utilisé dans la présente description comme une concentration dans l'air, estimée, mesurée généralement au cours du temps et pouvant être exprimée à un instant donné en partie par milliards (ppb).

Le dispositif de suivi permet de fournir des valeurs d'un ou plusieurs paramètres environnementaux et d'au moins une concentration dans l'air d'un composant de l'air peut être estimée au cours du temps sur la base de valeurs de paramètres environnementaux acquises et/ou reçues par le dispositif.

Dans la présente description, le terme composant désigne un composant de l'air comme un polluant gazeux ou un composé naturel de l'air. Le terme concentration signifie une concentration dans l'air, par exemple volume/volume pour un gaz, masse/volume pour un solide ou encore en partie par million (ppm) ou milliard (ppb). L'environnement est défini comme étant l'environnement du dispositif selon la présente invention ou de l'utilisateur de ce dispositif.

L'environnement peut être un environnement intérieur, comme un espace clos de type logement, un environnement extérieur comme une rue ou un parc ou bien un environnement mixte comme une cours intérieure. Un paramètre environnemental est un paramètre qui affecte cet environnement, en particulier un paramètre lié à une activité humaine ou naturelle dans l'environnement. Ainsi, un paramètre environnemental peut être lié à la composition de l'air de l'environnement, comme une concentration d'un gaz, à un climat ou une météo comme un niveau d'ensoleillement ou encore à une activité humaine comme un taux d'occupation d'un logement.

Dans le cas d'un environnement intérieur, un paramètre environnemental peut avoir une valeur différente pour l'environnement intérieur et pour l'environnement extérieur qui l'entoure. Par exemple, il peut s'agir de niveaux de luminosité dans une pièce et à l'extérieur de cette pièce.

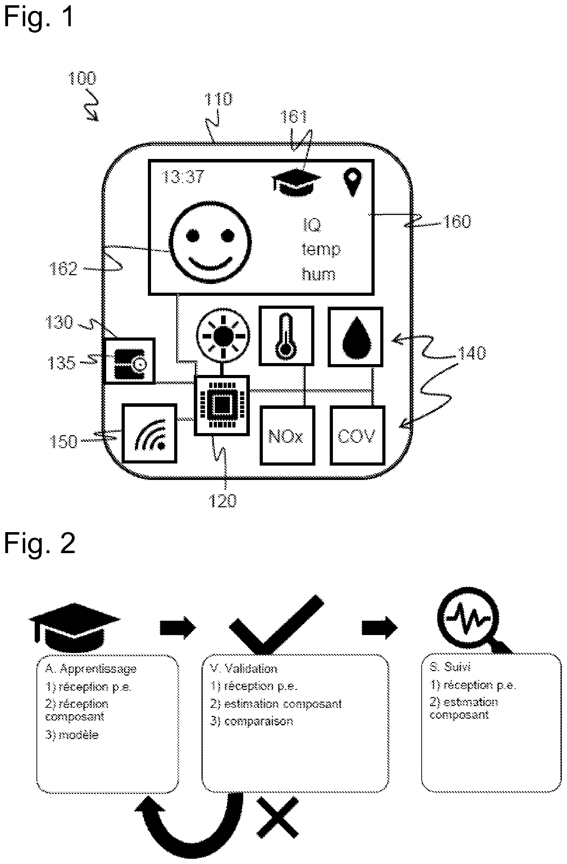

La figure 1 représente un dispositif 100 de suivi d'un composant de l'air. Ce dispositif de suivi 100 comprend un châssis 110 et un calculateur 120 logé dans le châssis 110. En outre, le dispositif 100 comprend un logement 130 pour un capteur consommable, un ensemble de capteurs 140 environnementaux et une interface de communication 150 connectés électroniquement au calculateur 120, par exemple via des câbles ou des pistes sur un circuit imprimé.

Un écran 160 tactile ou non peut être pourvu sur le dispositif 110 de manière à constituer une interface utilisateur. En outre, un ou plusieurs boutons physiques ou tactiles peuvent être placés sur le châssis 110 (non représentés).

Le calculateur 120 peut être de tout type adapté à l'apprentissage artificiel et par exemple comprendre un ou plusieurs noyaux neuronaux. On peut ainsi citer les processeurs Intel Movidius Myriad X et Texas Instruments Inc. TDA4VM. En outre, le calculateur 120 peut comprendre un processeur principal dédié aux taches d'échanges de données, de stockage, d'affichage et de communication et un coprocesseur dédié à l'apprentissage artificiel, comme par exemple le KL520 commercialisé par la société Kneron Inc.

L'interface de communication 150 peut comprendre une interface filaire comme USB et/ou préférentiellement une interface sans fil de type 4G, 5G, Wifi^{®}, Bluetooth^{®} ou encore ZigBee^{®}. L'interface de communication peut être intégré au calculateur 120 et peut comprendre une ou plusieurs antennes (non représentées).

Dans l'exemple de la figure 1, les capteurs environnementaux comprennent un capteur de luminosité, un capteur de température, un capteur d'humidité, un capteur d'oxyde d'azote et un capteur (COV). Par exemple, le capteur de composés organiques volatils est un capteur du type à photoionisation (PID), électrochimique, MOX ou encore photoacoustique.

Cependant, tout type de paramètres environnementaux peuvent être acquis par la pluralité de capteurs 140 du dispositif 100, et par exemple une température, un niveau de luminosité, un niveau de bruit, un degré d'humidité, une pression atmosphérique, un indice de réfraction de l'air, une concentration d'ozone, une concentration de dioxyde de carbone, une concentration de monoxyde de carbone, une concentration d'oxyde(s) d'azote, une concentration d'oxyde de soufre, une concentration d'ammoniaque, une concentration de méthane, une concentration de composés organiques volatiles, une concentration de particules et une position géographique.

En outre, un capteur consommable 135 dédié à un composant de l'air spécifique, peut être inséré dans le logement 130 et le dispositif 100 peut alors réaliser une phase d'apprentissage, comme expliqué ci-après. Préférentiellement, le capteur consommable 135 est un capteur spécifique d'un ou plusieurs composés organiques volatils. Encore préférentiellement, le capteur consommable 135 est spécifique au formaldéhyde, et par exemple est du type décrit dans le document FR3050270B1.

Par exemple, le capteur consommable est d'un type différent du capteur de composés organiques volatils ou différent de tout autre capteur du dispositif. Le capteur consommable 135 a une durée de vie limitée, par exemple par une quantité cumulée de composant de l'air mesurée et/ou sur un temps déterminé par exemple, 1 semaine, 10 jours, 2 semaines ou plus.

La figure 2 représente une méthode de suivi selon la présente invention, pouvant être mis en œuvre par le calculateur 120 du dispositif 100.

Dans une phase d'apprentissage A, le capteur consommable 135 est présent dans le logement 130 et l'écran 160 peut afficher une icône dédiée 161 (voir Fig. 1)

Dans une étape 1), le calculateur reçoit des valeurs des paramètres environnementaux acquises par exemple à partir de la pluralité de capteurs 140 et/ou à partir de capteurs distants au travers de l'interface de communication 150. Ainsi, l'interface de communication 150 peut être en relation avec des serveurs dans le nuage fournissant des valeurs de paramètres environnementaux obtenues par un réseau public ou privé de capteurs environnementaux et/ou par des modèles de pollution de l'air ou des modèles météorologiques.

Par exemple, les capteurs environnementaux 140 du dispositif 100 permettent d'acquérir des valeurs de paramètres environnementaux d'un environnement intérieur et l'interface de communication 150 permet d'obtenir des valeurs de paramètres environnementaux d'un environnement extérieur.

En outre, l'interface de communication 150 peut permettre d'obtenir des valeurs de paramètres environnementaux de l'environnement intérieur ou extérieur à partir d'autres dispositifs domotique situés dans le même environnement que le dispositif 100, par exemple dans un même logement.

Par exemple, il peut s'agir d'une valeur de réglage d'un thermostat, d'une valeur d'une température extérieure, de la position de volets électriques, de la présence d'une ou de plusieurs personnes dans l'environnement, de la consommation de données d'un réseau sans fil ou d'une passerelle internet, de la consommation électrique du logement ou de certains appareils électriques de l'environnement, de la position ouverte ou fermée d'une ou plusieurs portes ou de la production de panneaux solaires situés sur un toit du logement.

Enfin, l'utilisateur peut être invité à déclarer des valeurs de paramètres environnementaux via l'interface utilisateur comme l'écran 160 du dispositif 100, un ordinateur, un téléphone intelligent ou une tablette tactile. Par exemple, il peut être invité à renseigner une adresse ou une position géographique de l'environnement dans lequel le dispositif 100 est disposé, le type d'environnement (intérieur, extérieur ou mixte), la nature de e l'environnement (appartement, maison, garage, bureau, parc, rue), une surface ou des dimensions de l'environnement, le nombre d'occupants du logement, l'âge des occupants du logement, la présence, le nombre et la nature d'animaux domestiques, une date de rénovation des peintures ou encore la présence de travaux dans le logement ou à proximité

Dans une étape 2), simultanée ou concomitante à l'étape 1), le calculateur reçoit des concentrations du composant de l'air mesurées par le capteur consommable 135. Par exemple, il s'agit de valeurs instantanées ou cumulées de la concentration du composant dans l'air ambient, comme l'air d'un environnement intérieur.

Dans une étape 3), un modèle de concentration dudit composant est établi par le calculateur 120. Ce modèle comprend l'établissement d'une corrélation entre les valeurs de concentration du composant acquise par le capteur consommable 135 et les valeurs des paramètres environnementaux acquises par la pluralité de capteur 140 et/ou réceptionnées au travers de l'interface de communication 150. En outre, ce modèle peut prendre en compte l'heure et la date des mesures, par exemple via une horloge interne du calculateur 120, via des valeurs temporelles renseignées par l'utilisateur dans l'interface utilisateur et/ou via des valeurs temporelles reçues d'un serveur ou d'une horloge distante.

Préférentiellement, cette phase d'apprentissage est réalisée durant un temps déterminé, par exemple au moins sept jours, au moins 14 jours ou au moins 21 jours. Ce temps prédéterminé peut être égal ou inférieur é la durée de vie du capteur consommable 135.

Le modèle peut être établi en utilisant tout type de méthode d'apprentissage machine, par exemple un réseau de neurone, des arbres décisionnels, des forêts d'arbres décisionnels (comme XGBoost), une machine à vecteurs de supports (SVM), un réseau de neurones, par exemple un réseau de neurones récurrents (RNN) pouvant être du type mémoire à court-terme longue (LSTM) ou du type réseau récurrent à portes (GRN), ou bien tout autre type de méthode ou moyen d'apprentissage machine. Le but de ce modèle est de pouvoir prédire ou estimer une valeur de concentration du composant de l'air en l'absence du capteur consommable 135.

Dans une phase de validation V optionnelle (voir Figure 2), le modèle établi est validé au regard des concentrations du composant mesurée par le capteur consommable 135, qui doit donc être encore fonctionnel.

Ainsi, dans une étape 1), des valeurs des paramètre environnementaux sont reçues par le calculateur 120. Dans une étape 2) ces valeurs de paramètres environnementaux sont fournies au modèle établi de manière à estimer une concentration du composant de l'air. Dans une étape 3), la concentration estimée du composant est comparée avec la concentration acquise par le capteur consommable 135.

Si la différence entre les concentrations acquise et estimées du composant ne dépasse pas un seuil de validité prédéterminé, alors le modèle établi est validé et le calculateur peut passer à l'étape de suivi S.

Au contraire, si cette différence est égale ou dépasse le seuil de validité prédéterminé, alors la phase d'apprentissage peut être relancée automatiquement par le calculateur 120 ou bien l'utilisateur peut être invité à la relancer au travers de l'interface utilisateur. En outre, une série de phases d'apprentissage et de phases de validation peut être réalisée jusqu'à atteindre un seuil de validité prédéterminé, par exemple en enrichissant à chaque itération les valeurs des paramètres environnementaux utilisées pour la phase d'apprentissage par des valeurs additionnelles.

Alternativement ou en combinaison, plusieurs modèles différents peuvent être établis simultanément et le modèle le plus fiable, c'est-à-dire permettant d'obtenir des valeurs estimées du composant de l'air les plus proches des valeurs mesurées, peut être sélectionné dans la phase de validation.

Dans la phase de suivi S (voir Figure 2), le capteur consommable peut ne plus être utilisé et la concentration du composant est alors suivie au travers de l'estimation faite par le modèle établi. Cette étape peut être lancée après validation du modèle établi, après un temps déterminé ou après la fin de vie du capteur consommable 135.

Ainsi, dans une étape 1), des valeurs des paramètre environnementaux sont reçues par le calculateur 120. Dans une étape 2) ces valeurs de paramètres environnementaux sont fournies au modèle établi de manière à estimer une concentration du composant de l'air. Cette concentration du composant de l'air peut alors être affichée à un utilisateur au travers de l'interface utilisateur, en concentration, en valeur absolue, au travers d'un indice de qualité de l'air IQ et/ou au travers d'un pictogramme comme le pictogramme 162 visible sur la Figure 1.

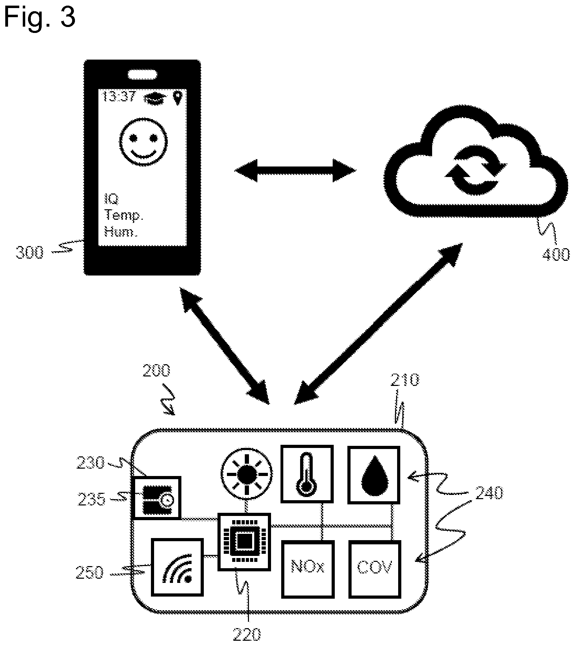

Dans un second mode de réalisation représenté sur la Figure 3, le dispositif 200 est plus simple que le dispositif 100 de la figure 1, en ce qu'il peut ne pas comprendre d'écran 160 et le calculateur 220 peut être d'un modèle simplifié comme un modèle incapable d'établir un modèle selon la présente invention. Dans ce cas, les valeurs des paramètres environnementaux acquises par la pluralité de capteurs 240 et les valeurs de concentration du composant acquise par le capteur consommable 235 sont transmises par le calculateur 220 simplifié au travers de l'interface de communication 250 à un dispositif informatique 300 de l'utilisateur comme un smartphone, une tablette tactile ou encore un ordinateur.

Le dispositif informatique 300 contient un calculateur adapté pour établir le modèle décrit précédemment, c'est à dire d'un niveau de puissance permettant un apprentissage machine. En outre, le modèle peut être établi par le dispositif informatique 300 sur la base de valeurs de paramètres environnementaux reçus à partir du nuage 400, comme décrit précédemment.

Enfin, le dispositif informatique 300 peut servir d'interface utilisateur de manière à permettre l'affichage des concentrations estimées du composant, comme décrit précédemment. De plus, cette interface utilisateur peut être utilisé pour acquérir de l'utilisateur certains paramètres environnementaux déclaratifs, de façon similaire au premier mode de réalisation.

Alternativement ou en combinaison, le calculateur permettant de mettre en œuvre le procédé décrit dans le premier mode de réalisation est hébergé dans le nuage 400, c'est-à-dire sur un serveur distant relié à Internet. Dans cette alternative, le dispositif informatique 300 peut ne servir qu'à l'interface utilisateur. Par exemple, le calculateur dans le nuage 400 peut réaliser l'établissement d'un ou de plusieurs modèles et le calculateur 300 peut mettre en œuvre le modèle validé.

Ainsi, le procédé de suivi du composant est similaire à celui décrit dans le premier mode de réalisation mais réalisé en dehors du dispositif de suivi 200. Ceci permet de proposer un dispositif de suivi 200 moins couteux ou plus petit, par un exemple un dispositif de suivi portable pouvant intégrer une batterie.

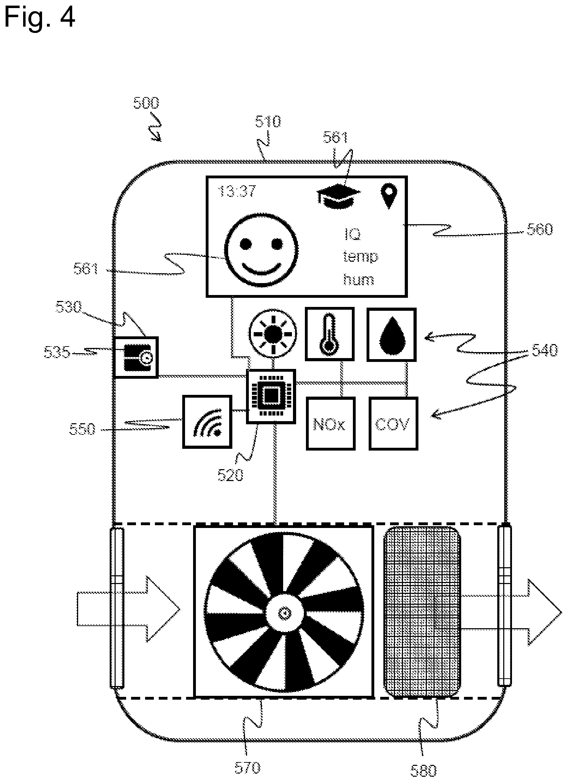

La figure 4 représente un troisième mode de résiliation dans lequel le dispositif 100 de suivi du composant de l'air selon le premier mode de réalisation est intégré dans un purificateur d'air ou appareil 500 de traitement de l'air. Cet appareil 500 de traitement de l'air comprend ainsi tous les éléments du dispositif 100 de la Figure 1 avec en plus un ventilateur 570 et un filtre 580 agencé pour générer et purifier un flux d'air. Préférentiellement, le filtre 580 est adapté pour retenir le composant de l'air de manière à purifier l'air d'un environnement intérieur.

Un procédé de suivi du composant de l'air identique à celui décrit dans le premier mode de réalisation peut être mis en œuvre par le calculateur 520. Cependant, les vitesses de rotation du ventilateur au cours de la période d'apprentissage ainsi qu'une durée d'utilisation du filtre peuvent être pris en compte lors de l'établissement du modèle.

En outre, la concentration estimée du composant peut être utilisée pour l'asservissement du ventilateur 570 et donc pour l'asservissement du traitement de l'air. Un tel appareil de traitement de l'air permet à un utilisateur de se protéger efficacement contre le composant de l'air même en l'absence du capteur consommable 235.

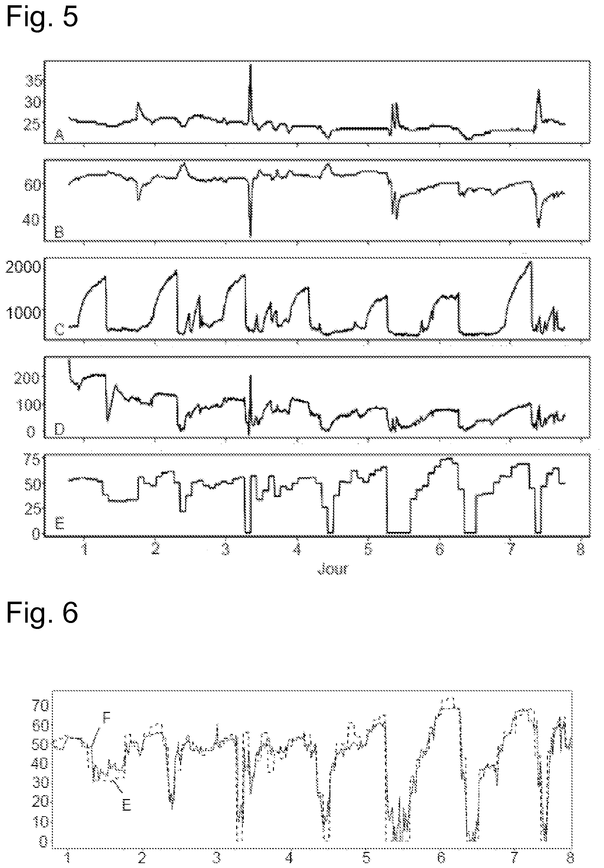

Dans un exemple de réalisation de la présente invention, une phase d'apprentissage a été réalisée durant 8 jours dans un environnement de test. La Figure 5 représente les valeurs des paramètres environnementaux suivant qui ont été acquis au cours du temps à partir de capteurs environnementaux placés dans un environnement intérieur déterminé : température de l'air (Courbe A en °C), humidité relative (Courbe B en %), concentration en dioxyde de carbone (Courbe C en ppb) et concentration en composé organique volatils légers (Courbe D en ppb), c'est-à-dire jusqu'à cinq atomes de carbone.

De plus des valeurs de concentrations de formaldéhyde (Courbe E en ppb) dans ce même environnement ont été acquises dans le même temps à partir d'un capteur du type mentionné précédemment.

La phase d'apprentissage a été établie sur la base d'un modèle de type arbres de décision (XGBoost) et des valeurs acquises visibles à la Figure 5.

La Figure 6 représente une phase de validation réalisée après la phase d'apprentissage de la Figure 5, de manière à comparer la courbe E représentant les valeurs de concentration acquises par le capteur consommable et utilisées pour la phase d'apprentissage avec la courbe F des valeurs de concentration estimées par le modèle, les valeurs étant en ppb.

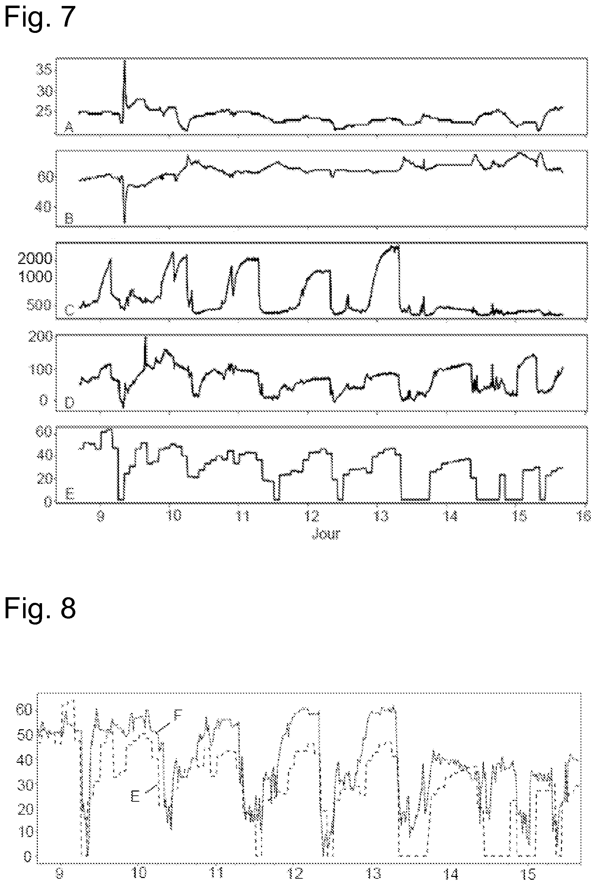

Dans une deuxième phase de validation, le modèle établi est alimenté avec de nouvelles valeurs des paramètres environnementaux mentionnés ci-dessus, également sur une durée de huit jours, comme visible sur la Figure 7. De façon similaire à la Figure 5, la courbe A représente des valeurs au cours du temps de la température de l'air (en °C), la courbe B des valeurs d'humidité relative (en %), la courbe C des concentrations en dioxyde de carbone (en ppb) et la courbe D des valeurs de concentration en composé organique volatils légers (en ppb), c'est-à-dire jusqu'à cinq atomes de carbone.

La courbe E représente à nouveau des concentrations en formaldéhyde mesurées par un capteur consommable, sur la même période de temps et à des fins de validation.

Ces nouvelles valeurs des paramètres environnementaux (courbes A à D) sont à nouveau soumises au modèle établi afin de générer des valeurs de concentrations estimées en formaldéhyde.

La Figure 8 représente une comparaison entre les valeurs mesurées de concentration en formaldéhyde (courbe E, en ppb) avec les valeurs estimées par le modèle établi (courbe F, en ppb).

On constate alors un bon niveau de corrélation entre les valeurs estimées et les valeurs acquise. En particulier, la forme de la courbe des valeurs estimées (courbe F) est proche de la forme de la courbe des valeurs acquises de concentration de formaldéhyde (courbe E) et reste dans le même ordre de grandeur. De plus, la courbe F des valeurs estimées de concentration en formaldéhyde reproduit assez fidèlement les variations de concentration du formaldéhyde dans l'environnement de test (courbe F).

Le modèle établi est donc validé et peut alors servir à estimer la concentration en formaldéhyde au cours du temps, en l'absence de capteur consommable spécifique au formaldéhyde.

On comprendra que diverses modifications et/ou améliorations évidentes pour l'homme du métier peuvent être apportées aux différents modes de réalisation de l'invention décrits dans la présente description sans sortir du cadre de l'invention défini par les revendications annexées. En particulier, il est fait référence aux capteur environnementaux qui peuvent comprendre tout type de capteur, au boitier du dispositif qui peut prendre toute forme de dispositif portable ou résidentiel.

En outre, les caractéristiques des différents modes de réalisation peuvent être échangées. Ainsi, le dispositif de la figure 1 ou l'appareil de traitement de l'air selon la figure 4 peuvent avoir un calculateur et/ou un écran déporté de façon similaire au dispositif de la figure 3, en alternative ou en combinaison avec les écrans 160, 560 et les calculateurs 120, 520.

## Revendications

1. Dispositif de suivi (100, 200) d'un composant de l'air,
le dispositif comprenant une pluralité de capteurs (140, 240) configurés pour acquérir des valeurs de paramètres environnementaux,
**caractérisé en ce que** le dispositif (100, 200) comprend en outre :
- un capteur consommable (135, 235) configuré pour acquérir des valeurs de concentration dudit composant, le capteur consommable (135, 235) ayant une durée de vie prédéterminée et
- un calculateur (120, 220) configuré pour :
• dans une phase d'apprentissage, établir un modèle de la concentration dudit composant en établissant une corrélation entre des valeurs de concentration dudit composant acquises par le capteur consommable (135, 235) et des valeurs des paramètres environnementaux acquises par la pluralité de capteurs (140, 240) et
• dans une phase de suivi, estimer la concentration dudit composant en utilisant le modèle établi et des valeurs des paramètres environnementaux acquises par la pluralité de capteurs (140, 240),
le dispositif de suivi (100, 200) comprenant un châssis (110, 210) dans lequel le calculateur (120, 220) est logé et un logement (130, 230) pour le capteur consommable (135, 235).

2. Dispositif (100, 200) selon la revendication 1, **caractérisé en ce que** le calculateur (120, 220) est configuré pour réaliser la phase de suivi au moins à partir du moment où la durée de vie du capteur consommable (135, 235) est expirée ou à partir du moment où le capteur consommable (135, 235) est déconnecté du dispositif (100, 200).

3. Dispositif (100, 200) selon la revendication 1 ou 2, **caractérisé en ce que** le calculateur (120, 220) est en outre configuré pour, dans une phase de validation, comparer des valeurs estimées de concentration du composant à des valeurs de concentration du composant acquises par le capteur consommable (135, 235).

4. Dispositif (100, 200) selon la revendication 3, **caractérisé en ce que** le calculateur (120, 220) est en outre configuré pour démarrer une nouvelle phase d'apprentissage ou pour envoyer un message à un utilisateur l'informant de la nécessité de démarrer une nouvelle phase d'apprentissage, si un écart entre des valeurs de concentration du composant estimées et des valeurs de concentration du composant acquises égale ou dépasse un seuil de validité prédéterminé du modèle établi durant la phase de validation.

5. Dispositif (100, 200) selon l'une des revendications 1 à 4, comprenant en outre une interface utilisateur et dans lequel le calculateur (120, 220) est configuré pour attribuer une durée de vie au modèle établi et, quand la durée de vie du modèle préétabli est expirée, pour démarrer une nouvelle phase d'apprentissage ou pour envoyer un message à un utilisateur sur l'interface utilisateur l'informant de la nécessité de démarrer une nouvelle phase d'apprentissage.

6. Dispositif (100, 200) selon l'une des revendications 1 à 4, comprenant en outre un capteur de position ou une interface utilisateur permettant à l'utilisateur de renseigner une position du dispositif (100, 200), et dans lequel le calculateur (120, 220) est configuré pour démarrer une nouvelle phase d'apprentissage en cas de changement de position dudit dispositif (100, 200), ou pour envoyer un message à un utilisateur sur l'interface utilisateur l'informant de la nécessité de démarrer une nouvelle phase d'apprentissage.

7. Dispositif (100, 200) selon l'une des revendications 1 à 6, le dispositif (100, 200) comprenant en outre une interface de communication (150, 250) configurée pour recevoir des valeurs des paramètres environnementaux et le calculateur (120, 220) est configuré pour, dans la phase d'apprentissage, établir le modèle en établissant également une corrélation avec les valeurs des paramètres environnementaux reçues et, dans la phase de suivi, estimer la concentration dudit composant en utilisant également les valeurs des paramètres environnementaux reçues.

8. Dispositif (100, 200) selon l'une des revendications 1 à 7, **caractérisé en ce que** le composant de l'air est le formaldéhyde.

9. Dispositif (100, 200) selon l'une des revendications 1 à 8, **caractérisé en ce que** les paramètres environnementaux sont choisis parmi une température, un niveau de luminosité, un niveau de bruit, un degré d'humidité, une pression atmosphérique, un indice de réfraction de l'air, une concentration d'ozone, une concentration de dioxyde de carbone, une concentration de monoxyde de carbone, une concentration d'oxyde(s) d'azote, une concentration d'oxyde de soufre, une concentration d'ammoniaque, une concentration de méthane, une concentration de composés organiques volatiles, une concentration de particules et une position géographique.

10. Appareil (500) de traitement de l'air comprenant un dispositif (100, 200) de suivi de composant de l'air selon l'une des revendications 1 à 9.

11. Appareil (500) de traitement de l'air selon la revendication 10, dans lequel un calculateur (520) est en outre configuré pour contrôler un paramètre de traitement de l'air en fonction de la concentration estimée du composant.

12. Procédé d'affichage de concentrations d'un composant de l'air, par un dispositif selon l'une des revendications 1 à 9, comprenant :
a) Une phase d'apprentissage incluant les étapes de :
i. acquisition de valeurs de paramètres environnementaux par la pluralité de capteurs (140, 240),
ii. acquisition de valeurs de concentration dudit composant par un capteur consommable (135, 235) spécifique au composant, le capteur consommable (135, 235) ayant une durée de vie prédéterminée,
iii. communication des valeurs acquises des paramètres environnementaux et de valeurs de concentration dudit composant à un calculateur (120, 220) distant configuré pour établir un modèle de la concentration dudit composant en établissant une corrélation entre les valeurs de concentration dudit composant et les valeurs des paramètres environnementaux réceptionnées,
b) Une phase de suivi du composant de l'air comprenant les étapes de :
i. acquisition de valeurs de paramètres environnementaux par la pluralité de capteurs (140, 240),
ii. communication des valeurs acquises de paramètres environnementaux au calculateur (120, 220) distant,
iii. réception et affichage de valeurs estimées de la concentration du composant sur la base des valeurs acquises des paramètres environnementaux et du modèle établi à partir du calculateur (120, 220) distant.

## Patentansprüche

1. Überwachungsvorrichtung (100, 200) für eine Luftkomponente,
wobei die Vorrichtung eine Vielzahl von Sensoren (140, 240) umfasst, die so eingerichtet sind, dass sie Werte von Umgebungsparametern erfassen,
**dadurch gekennzeichnet, dass** die Vorrichtung (100, 200) ferner Folgendes umfasst:
- einen Verbrauchsmaterialsensor (135, 235), der so eingerichtet ist, dass er Konzentrationswerte der Komponente erfasst, wobei der Verbrauchsmaterialsensor (135, 235) eine vorbestimmte Lebensdauer aufweist, und
- einen Rechner (120, 220), der für Folgendes eingerichtet ist:
• in einer Lernphase, Erstellen eines Modells der Konzentration der Komponente, indem eine Korrelation zwischen den vom Verbrauchsmaterialsensor (135, 235) erfassten Konzentrationswerten der Komponente und den von der Vielzahl von Sensoren (140, 240) erfassten Werten der Umgebungsparameter erstellt wird, und
• in einer Überwachungsphase, Schätzen der Konzentration der Komponente, indem das erstellte Modell und die
von der Vielzahl von Sensoren (140, 240) erfassten Werte der Umgebungsparameter verwendet werden, wobei die Überwachungsvorrichtung (100, 200) ein Gehäuse (110, 210), in dem der Rechner (120, 220) untergebracht ist, und eine Aufnahme (130, 230) für den Verbrauchsmaterialsensor (135, 235) umfasst.

2. Vorrichtung (100, 200) nach Anspruch 1, **dadurch gekennzeichnet, dass** der Rechner (120, 220) so eingerichtet ist, dass er die Überwachungsphase zumindest ab dem Zeitpunkt durchführt, an dem die Lebensdauer des Verbrauchsmaterialsensors (135, 235) abgelaufen ist, oder ab dem Zeitpunkt, an dem der Verbrauchsmaterialsensor (135, 235) von der Vorrichtung (100, 200) getrennt wird.

3. Vorrichtung (100, 200) nach Anspruch 1 oder 2, **dadurch gekennzeichnet, dass** der Rechner (120, 220) ferner so eingerichtet ist, dass er in einer Validierungsphase geschätzte Konzentrationswerte der Komponente mit vom Verbrauchsmaterialsensor (135, 235) erfassten Konzentrationswerten der Komponente vergleicht.

4. Vorrichtung (100, 200) nach Anspruch 3, **dadurch gekennzeichnet, dass** der Rechner (120, 220) ferner so eingerichtet ist, dass er eine neue Lernphase startet oder eine Nachricht an einen Benutzer sendet, die ihn darüber informiert, dass eine neue Lernphase gestartet werden muss, wenn eine Abweichung zwischen geschätzten Konzentrationswerten der Komponente und erfassten Konzentrationswerten der Komponente gleich einem vorbestimmten Gültigkeitsschwellenwert des während der Validierungsphase erstellten Modells ist oder diesen überschreitet.

5. Vorrichtung (100, 200) nach einem der Ansprüche 1 bis 4, ferner umfassend eine Benutzeroberfläche und wobei der Rechner (120, 220) so eingerichtet ist, dass er dem erstellten Modell eine Lebensdauer zuweist und, wenn die Lebensdauer des vorerstellten Modells abgelaufen ist, eine neue Lernphase startet oder eine Nachricht an einen Benutzer auf der Benutzeroberfläche sendet, die ihn darüber informiert, dass eine neue Lernphase gestartet werden muss.

6. Vorrichtung (100, 200) nach einem der Ansprüche 1 bis 4, ferner umfassend
einen Positionssensor oder eine Benutzeroberfläche, die es dem Benutzer ermöglicht, eine Position der Vorrichtung (100, 200) einzugeben, und wobei der Rechner (120, 220) so eingerichtet ist, dass er eine neue Lernphase startet, wenn sich die Position der Vorrichtung (100, 200) ändert, oder eine Nachricht an einen Benutzer auf der Benutzeroberfläche sendet, die ihn darüber informiert, dass eine neue Lernphase gestartet werden muss.

7. Vorrichtung (100, 200) nach einem der Ansprüche 1 bis 6, wobei die Vorrichtung
(100, 200) ferner eine Kommunikationsschnittstelle (150, 250) umfasst, die so eingerichtet ist, dass sie Werte der Umgebungsparameter empfängt, und der Rechner (120, 220) so eingerichtet ist, dass er in der Lernphase das Modell erstellt, indem auch eine Korrelation mit den empfangenen Werten der Umgebungsparameter erstellt wird, und er in der Überwachungsphase die Konzentration der Komponente schätzt, indem auch die empfangenen Werte der Umgebungsparameter verwendet werden.

8. Vorrichtung (100, 200) nach einem der Ansprüche 1 bis 7, **dadurch gekennzeichnet, dass** die Luftkomponente Formaldehyd ist.

9. Vorrichtung (100, 200) nach einem der Ansprüche 1 bis 8, **dadurch gekennzeichnet, dass** die Umgebungsparameter aus einer Temperatur, einer Helligkeitsstufe, einem Lärmpegel, einem Feuchtigkeitsgrad, einem Luftdruck, einem Luftbrechungsindex, einer Ozonkonzentration, einer Kohlendioxidkonzentration, einer Kohlenmonoxidkonzentration, einer Stickoxidkonzentration, einer Schwefeloxidkonzentration, einer Ammoniakkonzentration, einer Methankonzentration, einer Konzentration flüchtiger organischer Verbindungen, einer Partikelkonzentration und einer geografischen Lage ausgewählt sind.

10. Luftaufbereitungsgerät (500), das eine Überwachungsvorrichtung (100, 200) für eine Luftkomponente nach einem der Ansprüche 1 bis 9 umfasst.

11. Luftaufbereitungsgerät (500) nach Anspruch 10, wobei ein Rechner (520) ferner so eingerichtet ist, dass er einen Luftaufbereitungsparameter in Abhängigkeit von der geschätzten Konzentration der Komponente steuert.

12. Verfahren zur Anzeige von Konzentrationen einer Luftkomponente durch eine Vorrichtung nach einem der Ansprüche 1 bis 9, umfassend:
a) eine Lernphase, die die folgenden Schritten enthält:
i. Erfassen von Werten von Umgebungsparametern durch die Vielzahl von Sensoren (140, 240),
ii. Erfassen von Konzentrationswerten der Komponente durch einen komponentenspezifischen Verbrauchsmaterialsensor (135, 235), wobei der Verbrauchsmaterialsensor (135, 235) eine vorbestimmte Lebensdauer aufweist,
iii. Übermitteln der erfassten Werte der Umgebungsparameter und der Konzentrationswerte der Komponente an einen entfernten Rechner (120, 220), der so eingerichtet ist, dass er ein Modell der Konzentration der Komponente erstellt, indem eine Korrelation zwischen den Konzentrationswerten der Komponente und den empfangenen Werten der Umgebungsparameter erstellt wird,
b) eine Überwachungsphase für die Luftkomponente, die die folgenden Schritte umfasst:
i. Erfassen von Werten von Umgebungsparametern durch die Vielzahl von Sensoren (140, 240),
ii. Übermitteln der erfassten Werte von Umgebungsparametern an den entfernten Rechner (120, 220),
iii. Empfangen und Anzeigen von geschätzten Werten der Konzentration der Komponente auf der Grundlage der erfassten Werte der Umgebungsparameter und des von dem entfernten Rechner (120, 220) erstellten Modells.

## Claims

1. A device (100, 200) for monitoring a component of the air,
the device comprising a plurality of sensors (140, 240) configured to acquire values of environmental parameters,
**characterized in that** the device (100, 200) further comprises:
- a consumable sensor (135, 235) configured to acquire values of concentration of said component, the consumable sensor (135, 235) having a predetermined service life and
- a calculator (120, 220) configured to:
• in a learning phase, establish a model of the concentration of said component by establishing a correlation between values of concentration of said component acquired by the consumable sensor (135, 235) and values of the environmental parameters acquired by the plurality of sensors (140, 240) and
• in a monitoring phase, estimate the concentration of said component using the established model and values of the environmental parameters acquired by the plurality of sensors (140, 240),
the monitoring device (100, 200) comprising a chassis (110, 210) in which the calculator (120, 220) is housed and a housing (130, 230) for the consumable sensor (135, 235).

2. The device (100, 200) according to claim 1, **characterized in that** the calculator (120, 220) is configured to carry out the monitoring phase at least from the moment when the service life of the consumable sensor (135, 235) has expired or from the moment when the consumable sensor (135, 235) is disconnected from the device (100, 200).

3. The device (100, 200) according to claim 1 or 2, **characterized in that** the calculator (120, 220) is further configured to, in a validation phase, compare estimated values of concentration of the component with values of concentration of the component acquired by the consumable sensor (135, 235).

4. The device (100, 200) according to claim 3, **characterized in that** the calculator (120, 220) is further configured to start a new learning phase or to send a message to a user informing him of the need to start a new learning phase, if a difference between estimated values of concentration of the component and acquired values of concentration of the component equals or exceeds a predetermined validity threshold of the model established during the validation phase.

5. The device (100, 200) according to any of claims 1 to 4, further comprising a user interface and wherein the calculator (120, 220) is configured to assign a service life to the established model and, when the service life of the pre-established model has expired, to start a new learning phase or to send a message to a user on the user interface informing him of the need to start a new learning phase.

6. The device (100, 200) according to any of claims 1 to 4, further comprising a position sensor or a user interface allowing the user to enter a position of the device (100, 200), and wherein the calculator (120, 220) is configured to start a new learning phase in case of a change in the position of said device (100, 200), or to send a message to a user on the user interface informing him of the need to start a new learning phase.

7. The device (100, 200) according to any of claims 1 to 6, the device (100, 200) further comprising a communication interface (150, 250) configured to receive values of the environmental parameters and the calculator (120, 220) is configured to, in the learning phase, establish the model by also establishing a correlation with the received values of the environmental parameters and, in the monitoring phase, estimate the concentration of said component by also using the received values of the environmental parameters.

8. The device (100, 200) according to any of claims 1 to 7, **characterized in that** the component of the air is formaldehyde.

9. The device (100, 200) according to any of claims 1 to 8, **characterized in that** the environmental parameters are chosen from a temperature, a brightness level, a noise level, a degree of humidity, an atmospheric pressure, a refractive index of the air, a concentration of ozone, a concentration of carbon dioxide, a concentration of carbon monoxide, a concentration of nitrogen oxide(s), a concentration of sulfur oxide, a concentration of ammonia, a concentration of methane, a concentration of volatile organic compounds, a concentration of particles and a geographical position.

10. An apparatus (500) for treating air comprising a device (100, 200) for monitoring component of the air according to any of claims 1 to 9.

11. The apparatus (500) for treating air according to claim 10, wherein a calculator (520) is further configured to control a parameter for treating air according to the estimated concentration of the component.

12. A method for displaying concentrations of a component of the air, by a device according to any of claims 1 to 9, comprising:
a) a learning phase including the steps of:
i. acquiring values of environmental parameter by the plurality of sensors (140, 240),
ii. acquiring values of concentration of said component by a consumable sensor (135, 235) specific to the component, the consumable sensor (135, 235) having a predetermined service life,
iii. communicating the acquired values of the environmental parameters and values of concentration of said component to a remote calculator (120, 220) configured to establish a model of the concentration of said component by establishing a correlation between the values of concentration of said component and the received values of the environmental parameters,
b) a phase of monitoring the component of the air comprising the steps of:
i. acquiring values of environmental parameters by the plurality of sensors (140, 240),
ii. communicating the acquired values of environmental parameter to the remote calculator (120, 220),
iii. receiving and displaying estimated values of the concentration of the component based on the acquired values of the environmental parameter and the model established from the remote calculator (120, 220).
